(19) [Europäisches Patentamt / European Patent Office / Office européen des brevets]

(11) **EP 1 612 282 B1**

(12) **EUROPEAN PATENT SPECIFICATION**

(45) Date of publication and mention
of the grant of the patent:
**25.05.2011 Bulletin 2011/21**

(51) Int Cl.:
***C12Q 1/68*** (2006.01)

(21) Application number: **05254139.8**

(22) Date of filing: **30.06.2005**

(54) **Probe set and substrate for detecting nucleic acid**

Sondensatz und Substrat zum Nachweis von Nukleinsäuren

Ensemble de sondes destiné à la détection des acides nucléiques

(84) Designated Contracting States:
**DE FR GB**

(30) Priority: **02.07.2004 JP 2004197090**

(43) Date of publication of application:
**04.01.2006 Bulletin 2006/01**

(73) Proprietor: **CANON KABUSHIKI KAISHA**
**Tokyo 146 (JP)**

(72) Inventors:
• **Suzuki, Tomohiro**
**Ohta-ku, Tokyo (JP)**
• **Ishii, Mie**
**Ohta-ku, Tokyo (JP)**

(74) Representative: **Weser, Wolfgang et al**
**Weser & Kollegen**
**Patentanwälte**
**Radeckestrasse 43**
**81245 München (DE)**

(56) References cited:
**EP-A- 1 516 933     US-A1- 2001 053 526**
**US-B1- 6 410 229**

• HUBER M ET AL: "ACCESSING SINGLE
NUCLEOTIDE POLYMORPHISMS IN GENOMIC
DNA BY DIRECT MULTIPLEX POLYMERASE
CHAIN REACTION AMPLIFICATION ON
OLIGONUCLEOTIDE MICROARRAYS"
ANALYTICAL BIOCHEMISTRY, ACADEMIC
PRESS, NEW YORK, NY, US, vol. 303, no. 1, 1 April
2002 (2002-04-01), pages 25-33, XP001126279
ISSN: 0003-2697
• REYES-LOPEZ M A ET AL: "Fingerprinting of
prokaryotic 16S rRNA genes using
oligodeoxyribonucleotide microarrays and
virtual hybridization" NUCLEIC ACIDS
RESEARCH, OXFORD UNIVERSITY PRESS,
SURREY, GB, vol. 31, no. 2, 15 January 2003
(2003-01-15), pages 779-789, XP002306890 ISSN:
0305-1048

SEQUENCE LISTING

<110> CANON KABUSHIKIKAISHA

<120> PROBE SET AND CARRIER FOR DETECTING NUCLEIC ACID

<130> 2961130

<150> 5519291-01
<151> 2004-07-02

<160> 8

<170> Patentln version 3.3

<210> 1
<211> 24
<212> DNA
<213> Artificial sequence

<220>
<223> Primer for PCR

<400>
tgatttgggt gatggttcac gtag          24

<210> 2
<211> 22
<212> DNA
<213> Artificial sequence

<220>
<223> Primer for PCR

<400> 2
atcagcaata aaccagccag cc          22

<210> 3
<211> 60
<212> DNA
<213> Artificial sequence

<220>

<223> Primer for PCR

<400> 3
ttttatggtg cactctcagt acaatctgct ctgatgccgc atagttaagc cagccccgac        60

<210> 4
<211> 40
<212> DNA
<213> Artificial sequence

<220>
<223> Primer for PCR

<400> 4
agttgggtgc acgagtgggt tacatcgaac tggatctcaa        40

<210> 5
<211> 25
<212> DNA
<213> Artificial sequence

<220>
<223> Primer for PCR

<400> 5
gataaagttg caggaccact tctgc        25

<210> 6
<211> 25
<212> DNA
<213> Artificial sequence

<220>
<223> Primer for PCR

<400> 6
gcagattgta ctgagagtgc accat        25

<210> 7
<211> 25
<212> DNA
<213> Artificial sequence

<220>
<223> Primer for PCR

<400> 7
atggtgcact ctcagtacaa tctgc        25

<210> 8
<211> 25
<212> DNA
<213> Artificial sequence

<220>
<223> gtgggttaca tcgaactgga tctca

<400> 8

gtgggttaca tcgaactgga tctca    25

**Description**

The present invention relates to a probe set composed of a plurality of probes for detecting a nucleic acid to be detected. The present invention also relates to a probe substrate for detecting a nucleic acid, on which the plurality of probes are immobilized.

As typified by the human genome project, the gene sequences of a variety of organisms have been elucidated and the relationship between genes and the mechanisms of life activity, diseases, constitutions and so on has been investigated successively. It has been found out that the determination of the presence or absence of genes and the abundance of genes (the amount of genes expressed) allows, for example, the more detailed characterization or typing of diseases or the selection of effective therapies for diseases.

Many methods have heretofore been proposed for examining the presence or absence and the abundance of a particular gene contained in a sample. Particularly, a method that has a broad range of applications and is applicable regardless of subjects to be detected is a widely-used method in which a partial sequence characteristic of a gene or nucleic acid to be detected is selected to examine the presence or absence and the abundance of the partial sequence. Specifically, in the method, a nucleic acid (probe) having a sequence corresponding to a complementary strand of the selected partial sequence is prepared, and the hybridization of the probe with a sample is detected by means of some kind, thereby examining the presence or absence of the nucleic acid to be detected in the sample.

The method for detecting a particular nucleic acid that utilizes hybridization can be used in both solid and liquid phases. As an example of the method performed in a liquid phase, a probe solution containing a probe bound with a labeling substance that changes in spectral characteristics when the probe forms a double strand is prepared and supplemented with a sample, and a change in the spectral characteristics is measured, thereby examining the presence or absence of a particular nucleic acid in the sample

In a typical detection method using hybridization on a solid phase, a probe is immobilized or adsorbed on a solid phase which is in turn supplemented with a sample labeled with a certain detectable labeling substance, and a signal from the labeling substance on the solid phase is measured. Among them, for example, chips where probes are immobilized on a flat substrate (e.g., glass or metal) or beads where probes are immobilized on the surfaces of microparticles are typical forms of solid-phase hybridization. The solid-phase hybridization is preferably used for such reasons that: B/F (bound/free) separation is easily performed; a detection domain can be rendered physically minute and is therefore expected to be highly sensitive; multiple items can be detected simultaneously by placing a plurality of types of probes in physical isolation; and a solid phase can be easily handled and applied.

One of advantageous features of solid-phase hybridization, simultaneous detection of multiple items, allows various detection schemes. For example, when family genes having partially the same sequences are detected, one probe is assigned to a domain having a common sequence among family genes, while each one probe is assigned to each specific domain having a sequence differing among the family genes, thereby allowing the comparison of the amount of a gene expressed among a plurality of samples and the accurate determination of what type of a family gene is expressed. When a causative organism of an infectious disease is distinguished, generally, a partial sequence encoding 16S ribosomal RNA in the gene of the organism is detected to distinguish a microbial species based on the detected sequence. In this case, by selecting a specific or common sequence at the microbial species, strain or genus level, a determination of species at the desired level can be achieved. For example, a probe is designed for a domain common to the same microbial species but different in each of other microbial species, and at the same time; a probe is designed for a domain different in each of strains derived from the same species. Accordingly, these plurality of probes can be used to simultaneously distinguish the bacterial species and the strains.

One example of a DNA microarray in which a plurality of probes are assigned to an identical nucleic acid to be detected is the Affymetrix (USA) GeneChip. A detection method used by the Affymetrix GeneChip DNA microarray is performed in the following procedures: a labeled nucleic acid is allowed to act on oligo DNA synthesized on a flat substrate, and the hybridization of the nucleic acid with the oligo DNA is measured by fluorescent detection, thereby detecting the presence or absence and the amount of a particular nucleic acid contained in a sample (see U.S. Patent No. 6410229). Signals from approximately 10 to 20 types of probes assigned to an identical nucleic acid to be detected are collectively accessed to measure the amount of the gene expressed.

The paper by Miguel Ángel Reyes-López et al. entitled "Fingerprinting of prokaryotic 16S rRNA genes using oligodeoxyribonucleotide microarrays and virtual hybridization" in NUCLEIC ACIDS RESEARCH, 2003, Vol.31, No.2, pages 779-789 discloses equal-length probes designed for one Bacillus species and six *Pseudomonas* strains. DNA sequences near the 5' end of 16S rRNA genes were aligned and two contiguous regions of high variability, flanked by highly conserved sequences, were found. The conserved sequences were used to design PCR primers that efficiently amplified the polymorphic regions from all seven species.

The paper by Martin Huber et al. entitled "Accessing Single Nucleotide Polymorphisms in Genomic DNA by Direct Multiplex Polymerase Chain Reaction Amplification on Oligonucleotide Microarrays" in ANALYTICAL BIOCHEMISTRY, 2002, Vol.303, No.1, pages 25-33 discloses a DNA microarray-based genotype the system for accessing single nucleotide polymorphisms directly from a genomic DNA sample. A one-step approach is described which combines multiplex amplification and allele-specific solid-phase PCR into an on-chip reaction platform.

On the other hand, improvements have been made in various quarters to the method for detecting a nucleic acid to be detected by hybridization using a probe nucleic acid in both liquid and solid phases. Consequently, the detection sensitivity has been enhanced more significantly than ever before. However, the direct detection of a nucleic acid to be detected by hybridization requires a sample in very large amounts and as such, is often unpractical. The amount of a sample that is generally available is not sufficient for the method in terms of sensitivity. Besides, in general, the method requires the labeling of a nucleic acid to be detected with a fluorescent substance or the like. Therefore, in most cases, the nucleic acid to be detected is amplified by a PCR or RNA polymerase reaction method, labeled and then hybridized with a prepared probe nucleic acid.

The amplified nucleic acid to be detected and the probe nucleic acid form a hybrid. Because a probe is generally a nucleic acid that is synthesized artificially and chemically, its nucleotide length often ranges approximately 15 mer to 80 mer. In general, a nucleic acid to be detected that has been amplified by a PCR method or the like is 100 mer or more in length. Accordingly, a hybrid formed by the probe and the nucleic acid to be detected has a structure in which a probe-binding region forms a double strand portion and the single strand portion of the nucleic acid to be detected overhangs on at least one side of the probe-binding region.

SUMMARY OF THE INVENTION

A plurality of different probes designed to detect a plurality of different domains in a target single-stranded nucleic acid is effective in detecting family genes and in distinguishing a microbial species as previously illustrated.

However, the use of a probe set that can detect different domains in an identical nucleic acid to be detected leads to variations in the stability of hybrids formed in different probe-binding regions, and the same level of detection sensitivity for the individual hybrids across the nucleic acid to be detected could not be attained.

In order to solve this problem, those skilled in the art may attempt to design a plurality of different probes having the same level of binding strength by focusing on a Tm value and GC% (GC content, which means {(the number of a guanine (G) base)+(the number of a cytosine (C) base)}/(the total number of bases) (%) within oligonucleotide). However, the present inventors have obtained a finding that the design of a plurality of probes by focusing only on the Tm values or GC% thereof results in great variations in the stability of hybrids formed in probe-binding regions. In short, this alone can not solve a problem with fluorescent intensity that greatly varies among probe-binding regions in spite of the use of samples in equal amounts.

Thus, an embodiment of the present invention seeks to provide a method of detecting a single-stranded nucleic acid to be detected by probe with high sensitivity, in which dispersion in measured values resulting from the stability of hybrids between the probes and the single-stranded nucleic acid is eliminated and to provide a probe substrate formed using the probe set.

The present inventors have diligently studied for solving the above-described problem and found out that the problem of dispersion among a plurality of probes can be addressed by providing a probe set composed of probes with the same level of hybrid stability, which are designed with consideration given to the stability of hybrids between the probes and a nucleic acid to be detected.

The present inventors have further searched guidelines for the design of probes capable of achieving the same level of hybrid stability and found out the following solutions:

In a first aspect, the present invention provides the method according to claim 1. In a second aspect, the present invention provides the method according to claim 10. The other claims relate to further developments.

The present invention can provide a probe set for conducting detection in two or more domains of a nucleic acid to be detected, wherein the same level of hybrid stability is attained.

Specifically, a probe set composed of two or more probes assigned to an identical nucleic acid to be detected, which has the same level of hybrid stability can be obtained. Moreover, by assigning one or more probes to each of a nucleic acid to be detected and a complementary strand thereof, a probe set having the same level of hybrid stability can be obtained. Furthermore, a probe substrate bound with any of these probe sets can also be obtained. This allows the establishment of required and desired probes without concern for the stability of hybrids.

Other features and advantages of the present invention will be apparent from the following description taken in conjunction with the accompanying drawings, in which:

FIG. 1 is a diagram showing the relationship among L1, L2 and P when a probe is assigned to a nucleic acid to be detected;

FIG. 2 is a diagram when two types of probes differing in nucleotide length are assigned to a nucleic acid to be detected; and

FIG. 3 is a diagram when two types of probes are assigned to a nucleic acid to be detected and a complementary strand thereof.

Preferred embodiments of the present invention will now be described in detail in accordance with the drawings.

The present inventors have proceeded with researches on the stability of a hybrid having a partial double strand structure. The present applicant has already filed an application for the invention focusing on the stability as Japanese Patent Application No. 2003-324647. Those researches have revealed that the stability of a hybrid differs depending on the ratio of the nucleotide length of a 5'-side single strand portion/the nucleotide length of a 3'-side single strand portion of two single strand portions consisting of a nucleic acid to be detected, which flank both ends of the double strand portion of a hybrid. To be more specific, it has been revealed that, when the number of bases in a single strand portion including the 5' end of a strand to be detected of two single strand portions is represented by L1 and the number of bases in a single strand portion including the 3' end thereof is represented by L2, a hybrid is rendered stable if L1 and L2 has the following relationship:

$$L2 \geq L1.$$

It has also been found out that the larger a relative ratio L2/L1 gets, the more stable a hybrid becomes.

This finding concerning hybrid stability indicates that the value of fluorescent intensity obtained from solid-phase hybridization or the like differs between probes even when a plurality of probes are assigned to an identical nucleic acid to be detected and the same Tm value is set for each of the probes.

In the present invention, the binding strength of a probe with a nucleic acid to be detected is one factor for the design of a probe. A Tm value, a temperature at which a double strand is dissociated into two single strands, is generally used as a measure showing the binding strength of a double strand formed by complementary strands bound with each other, for example, a double strand formed by a probe sequence and a sequence to be detected. A Tm value can be obtained by actually preparing a probe sequence and a complementary strand thereof and conducting measurement under the same atmosphere as hybridization between a sample nucleic acid and a probe. Alternatively, a Tm value of a probe can also be determined by calculation. For calculating a Tm value, a variety of calculation methods using a salt concentration and so on in hybridization as a parameter have been proposed. One example of the calculation methods that are frequently used in general is the Nearest neighbor method by which Tm values can be calculated for probes to give a probe set composed of the probes having sequences determined based on the Tm values. An alternative calculation method that can be used is the Wallace method by which Tm values can be calculated for probes to give a probe set composed of the probes having sequences determined based on the Tm values. The GC% method can also be used by which Tm values can be calculated for probes to give a probe set composed of the probes having sequences determined based on the Tm values

A method of adjusting a Tm value for optimizing the binding strength of a probe includes a method of changing the number of bases in the probe and a method of changing the GC% of the probe, whereby probes can be adjusted in binding strength to give a probe set composed of the probes.

In the present invention, the binding strength of each probe is set according to the number of bases from the 5' end of a nucleic acid to be detected to the detection position of the probe. Specifically, each probe is designed according to the number of bases from the 5' end of a nucleic acid to be detected to the detection position of the probe

In general, a DNA is used as a probe designed to detect a nucleic acid in terms of the ease with the probe being synthesized. However, for example, a PNA (peptide nucleic acid) that receives attention in recent years can also be employed as a probe. The binding strength of a PNA probe can appropriately be set in the same way as a DNA probe to thereby compose the probe set of the present invention. A probe set in which DNA and PNA probes are mixed can also be used, if necessary.

The probe set and the probe substrate according to the present invention will be described more fully.

(First probe set)

The first probe set according to the present invention is particularly effective when probes are desired in a plurality of portions in the sequence of a nucleic acid to be detected. A hybrid having a partial double strand portion composed of a nucleic acid to be detected and a probe has single strand portions that flank both ends of the double strand portion. When a single strand portion located on the 5'-endside of the nucleic acid to be detected is designated as a strand A and a single strand portion located on the 3'-endside thereof is designated as a strand B, the stability of the hybrid greatly differs according to the ratio of the length of the strand A/the length of the stand B. When the number of bases in the strand A is designated as L1 and the number of bases in the strand B is designated as L2, a hybrid is rendered stable if L1 and L2 satisfy the following relationship:

L2≥L1.

Moreover, the stability of a hybrid is known to increase with increase in the value of L2/L1. When the position of each probe-binding region in a nucleic acid to be detected is represented by P (the number of bases from the 5' end of the nucleic acid to be detected to the binding region), the stability of the hybrid and the binding strength of the probe increase as the value of P gets smaller, that is, the value of L2/L1 gets larger.

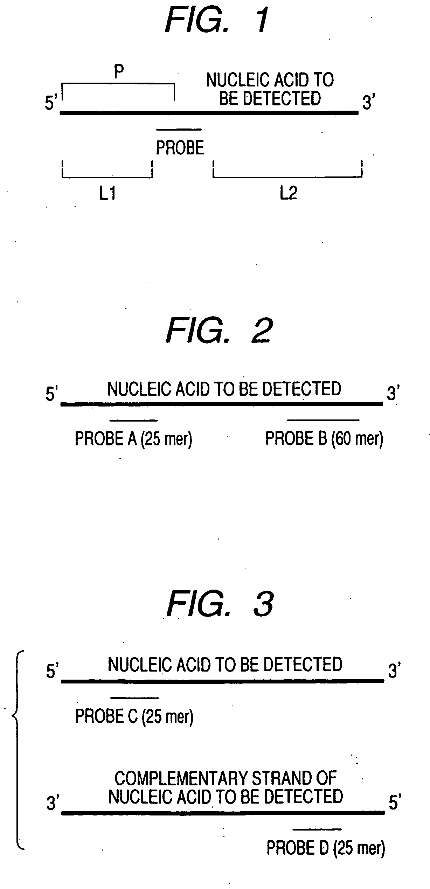

Thus, when a plurality of probes is assigned to a nucleic acid to be detected, a probe set composed of probes adjusted in binding strength according to the value of P is provided to thereby allow the same level of stability of hybrids formed by the probes and the nucleic acid to be detected. For reference, the relationship among L1, L2 and P is briefly shown in FIG. 1.

When probes composing a probe set are DNAs, the most general method of evaluating and representing its binding strength is a method employing a Tm value, a temperature at which a double strand is dissociated into two single strands. As described above, a Tm value can directly be measured by preparing a probe and a complementary strand thereof and subjecting them to absorbance measurement according to a standard method. In this measurement, it is preferred that the probes and the complementary strand thereof should be placed under conditions as similar as possible to actual hybridization conditions, that is, under conditions where a probe set and a sample nucleic acid containing a nucleic acid to be detected are hybridized. If measurement under the same conditions as actual hybridization conditions is difficult to attain, the measurement does not necessarily require those conditions and may be conducted under approximate conditions.

When a Tm value is not actually measured, it can also be determined by calculation. For calculating a Tm value, various approaches have been proposed, any of which can be used without limitation. However, particularly, the Nearest neighbor method, Wallace method and GC% method are preferably used in the probes encompassed by the present invention.

For the nearest neighbor method, see e.g., Breslauer K.J., Frank R., Blocker H., Markey L.A., Predicting DNA duplex stability from the nucleotide sequence, Proc. Natl. Acad. Sci. USA 83, 3746-3750 (1986); Freier S.M. Kierzek R., Jaeger J.A., Sugimoto N., Caruthers M.H., Nielson T., Turner D.H., Improved free-energy parameters for predictions of RNA duplex stability., Proc. Natl. Acad. Sci. USA 83, 9373-9377 (1986); Schildkraut C., Lifson S., Dependence of the melting temperature of DNA on salt concentration, Biopolymers 3, 195-208 (1965); for the Wallace method, see e.g., Wallace, R.B., Shaffer, J., Murphy R.F., Bonner, J., Hirose, T., Itakura, K. Nucleic Acids Res. 6, 3543 (1979); and for the GC% method, see e.g., Schildkraut C., Lifson S., Dependence of the Melting Temperature of DNA on Salt Concentration, BIOPOLYMERS 3. 195-208 (1965); W. Rychlik, Optimization of the annealing temperature for DNA amplification in vitro, Nucleic Acids Res. 18(21) 6409-6412 (1990).

When the Tm values of probes composing a probe set does not attain the desired values, it is required that a Tm value should be adjusted by changing the design of a sequence or the like in each of the probes. A method of adjusting a Tm value includes a method of changing the number of bases in the probe and a method of changing the GC% of the probe. Besides, a method in which a probe is subjected to a certain chemical modification to such an extent that specificity in sequence recognition is not significantly reduced can also be utilized.

In general, a DNA is used as a probe. However, a PNA is known to have the same function as a DNA. Thus, a PNA can be used as a probe by adjusting a Tm value in the same way as a DNA probe.

FIG. 2 shows one example according to the present invention in which a plurality of probes are assigned for a nucleic acid to be detected. In the probe set shown in FIG. 2, a probe A assigned on the 5'-endside of the nucleic acid to be detected is 25 mer in length, while a probe B assigned on the 3'-endside of the nucleic acid to be detected is 60 mer in length for enhancing binding strength.

(Second probe set)

The second probe set is a probe set for detecting a single-stranded nucleic acid to be detected and a single-stranded nucleic acid having a sequence complementary to the single-stranded nucleic acid to be detected, wherein one of the probes is capable of specifically binding to a first domain in the single-stranded nucleic acid to be detected and the other probe is capable of specifically binding to a second domain of the single-stranded nucleic acid having a complementary sequence, the first and second domains on the single-stranded nucleic acids satisfying the following positional relationship:

L1≤L2,

wherein the length of a nucleotide sequence in a domain other than the first and second domains on the 5'-endside of each of the single-stranded nucleic acids is designated as L1 and the length of a nucleotide sequence in a domain other than the first and second domains on the 3'-endside thereof is designated as L2.

When a sequence domain that requires detection is located on the 3'-endside of a nucleic acid to be detected (i.e., the value of L2/L1 is small), a hybrid formed by a probe and the nucleic acid to be detected has very low stability. In this case, the probe not only results in significant reduction in sensitivity as compared to other probes, but becomes incapable of detection if it falls short of detection sensitivity. In the second probe set, for avoiding this, a complementary strand of the nucleic acid to be detected can be used as another subject to be detected. That is, when the complementary strand is used as a subject to be detected, the values of L1 and L2 are reversed. As a result, the value of L2/L1 gets larger, and the probe and the strand to be detected (i.e., the complementary strand) can form a stable hybrid.

The assignment of a probe to a complementary strand can be applied particularly preferably when a sample nucleic acid is prepared by PCR. In a typical symmetric PCR method, a nucleic acid to be detected and a nucleic acid having a strand complementary to the nucleic acid to be detected are prepared in approximately equal amounts. A probe adaptable to a complementary strand can be used in detection by preparation of nucleic acid without making particular change in the procedures. Thus, the second probe set is a probe set in which at least one probe is designed for each of a nucleic acid to be detected and a complementary strand thereof with consideration given to hybrid stability.

Because a single-stranded nucleic acid to be detected requires amplification and labeling in most cases, it is often amplified and prepared in advance by a PCR method. In this case, a complementary strand of the single-stranded nucleic acid to be detected is also synthesized at the same time. Thus, the second probe set can be utilized particularly preferably when a PCR product is used as a subject to be detected. In the second probe set, at least one probe may be assigned to each of a nucleic acid to be detected and a complementary strand thereof. Two or more probes can also be assigned to an identical strand to be detected. The binding strength of each probe composing the probe set is determined according to the number of bases from the 5' end of a nucleic acid to be detected to a domain bound by the probe. Specifically, the probe is designed to have the relationship where the binding strength gets higher as the number of bases from the 5' end of a nucleic acid to be detected to a domain bound by the probe gets larger.

FIG. 3 shows an example of the second probe set when a plurality of probes are assigned to a nucleic acid to be detected. The probe set shown in FIG. 3 is composed of a probe C and a probe D for detecting two different domains in a strand to be detected, wherein the probe C assigned on the 5'-endside of the single DNA having a complementary sequence is 25 mer in length, while the probe D assigned on the 3'-endside of the strand to be detected is also 25 mer in length. Almost the same binding strength is set for these probes. In the design of the probes in the second probe set, the binding strength with a single-stranded nucleic acid can be set based on a Tm value in the same way as the first probe set.

Here, if a probe binding to a portion in a nucleic acid to be detected that corresponds to a domain recognized by the probe D.is designed, the relationship between L1 and L2 is given by L1≥L2, resulting in reduction in the stability of a formed hybrid. Therefore, a domain recognized by a probe is shifted to a complementary strand shown in FIG. 3, and the probe D is selected. Thereby, L1 and L2 satisfy L2≥L1 and the stability of a hybrid formed by the complementary strand and the probe n can therefore be secured. Thus, according to the second probe set, when different probes that recognize different domains in a strand to be detected are designed and a domain recognized by any of the probes is assigned to a domain on the 3'-endside of the strand to be detected, a probe-binding region is assigned to a complementary strand of the strand to be detected. Thereby, the stability of a hybrid with the probe that recognizes a domain on the 3'-endside of the strand to be detected can be secured without an increase in the binding strength of the probe, thus requirements for the design of a probe can be eased. It is noted that, in a probe assigned to a complementary strand, a domain recognized by the probe is assigned to the position where the value of L1/L2 ranges from 0 to 1.

(Probe substrate)

The probes composing at least one of the above-described first and second probe sets can be immobilized each individually on a solid phase to form a probe substrate. For example, the probe substrate can preferably utilized in the analysis of the family genes described above, in which one probe is assigned to a domain having a common sequence among the family genes, while each one probe is assigned to each specific domain having a sequence differing among the family genes. Thus, two or more types of probes composing each probe set are provided in a predetermined number, depending on an application purpose of analysis.

When immobilizing each probe composing a probe set onto a substrate, it is required that the type of each probe is distinguishable. For allowing the type of each probe to be distinguishable, various approaches including spatial, optical and temporal approaches can be used. For example, a DNA microarray in which probes are immobilized on a type-by-type basis on different areas kept separated on the same plane is a typical preferred example of the probe binding substrate of the present invention.

Various binding schemes such as adsorption, ionic bond, hydrogen bond and covalent bond can be applied to a method of immobilizing probes. For binding each probe by a preferable scheme, it is preferred that the desired functional group should be introduced into the probe which is in turn bound with the substrate via the functional group.

The introduction of a functional group as a fixing site to the 5' end of a probe nucleic acid is generally practiced with relative ease. Similarly, the introduction of a functional group as a fixing site to the 3' end of a probe nucleic acid is also generally practiced with relative ease. In addition, a probe can also be immobilized on a substrate via a functional group as a fixing site that is inserted into the sequence of the probe.

Any of those capable of immobilizing a probe thereon such as metal, glass, plastic, metal thin film and fiber can be used without particular limitation as a material for a solid phase substrate. Any form of a substrate such as planar, bead and strand substrates can be used without particular limitation.

One preferred example of a method of immobilizing a probe DNA includes a method disclosed in Japanese Patent Application Laid-Open No. H11-187900. The immobilization method disclosed therein is an approach where a maleimide group is introduced onto a glass substrate to which a probe DNA having the 5' end bound with a thiol group is then bound. By this binding manner, the probe DNA is immobilized onto the glass substrate as a solid phase substrate through a covalent bond. The types of probes are distinguished by immobilizing the probes on different area type-by-type

EXAMPLES

Hereinafter, the present invention will be described more fully with reference to Examples. However, Examples described below illustrate preferred embodiments according to the present invention and are not intended to limit the technical scope of the present invention.

(Example 1)

I. PCR of pUC118 EcoRI/BAP

(1) Design of primer

A vector pUC118 EcoRI/BAP (a total of 3162 bp in length) commercially available from Takara was selected as a model for a sample having a sequence to be tested. Based on its nucleotide sequence, a total of two types of primers, a forward primer F1 and a reverse primer R1, having sequences described below were designed. Information on the whole nucleotide sequence of pUC118 EcoRI/BAP is provided by Takara and is also available from a public database, etc

The primers were designed with consideration given to sequence, GC%, and melting temperature (Tm value) so that the desired partial nucleotide sequence in pUC118 EcoRI/BAP was amplified specifically and efficiently by PCR amplification. The Tm value was calculated on the conditions that: $Na^+$ is 50 mM; $Mg^{2+}$ is 1.5 mM; and a primer concentration is 0.5 $\mu$M.

Table 1

| SEQ ID No: | Designation | Sequence | Tm value |
|---|---|---|---|
| SEQ ID No.1 | F1 | 5' TGATTTGGGTGATGGTTCACGTAG 3' | 63.8˚C |
| SEQ ID No.2 | R1 | 5' ATCAGCAATAAACCAGCGAGCC 3' | 64.7˚C |

By using the above-described primers thus designed, and performing PCR amplification with pUC118 EcoRI/BAP as a template thereby, a PCR product of 1324 bp (PCR product 1) would be given.

(2) Synthesis of primer

Two types of primers designed in Example 1(1) were synthesized. Each primer was obtained by synthesizing a DNA strand having the designed nucleotide sequence with a DNA synthesizer according to a standard method. The synthesized DNA strands were purified by cartridge purification to obtain two types of primers. The obtained primers each were diluted with a TE buffer to 10 $\mu$M in concentration.

(3) PCR amplification reaction

Two types of primers synthesized in Example 1(2), the vector pUC118 EcoRI/BAP (manufactured by Takara) used as a template DNA, and a PCR kit HotStarTaq Master Mix (manufactured by QIAGEN) were used to perform PCR amplification reaction. The Master Mix includes four types of deoxynucleotides, dATP, dCTP, dTTP and dGTP. For fluorescently labeling a PCR product, Cy3dUTP (manufactured by Amersham Biosciences) was further added thereto to label the PCR product with Cy3.

PCR reaction conditions complied with the protocol described below, and a reaction solution having the composition shown in Table 2 below was prepared.

Table 2

| Component | Composition |
|---|---|
| Master Mix (manufactured by QIAGEN) | 25 μl |
| Template DNA (diluted pUC118 manufactured by Takara) | 1 μl (10 ng) |
| Forward Primer (F1) | 2.5 μl (25 pmol/tube) |
| Reverse Primer(R1) | 2.5 μl (25 pmol/tube) |
| Cy3 dUTP(1 mM) | 2 μl (25 pmol/tube) |
| Water | 17 μl |
| Total | 50 μl |

The prepared reaction solution was subjected to PCR amplification reaction using a commercially-available thermal cycler according to the temperature cycle protocol shown in Table 3 below: the reaction solution was held at 95°C for 15 minutes (for enzyme activation); in 25 cycles each having denaturation process at 92°C for 15 seconds, annealing process at 55°C for 30 seconds and extension process at 72°C for 60 seconds; and finally at 72°C for 10 minutes

Table 3

| Step | Process | Temperature | Duration |
|---|---|---|---|
| 1 | Enzyme activation | 95°C | 15 minutes |
| 2 | Denaturation | 92°C | 15 seconds |
| 3 | Annealing | 55°C | 30 seconds |
| 4 | Extension | 72°C | 60 seconds |
| 5 | Extension | 72°C | 10 minutes |
| * A cycle from step 2 to step 4 is repeated 25 times. | | | |

After the completion of amplification reaction, a PCR amplification product 1 was purified using a purification column (Qiagen QIAquick PCR Purification Kit). Following purification, the volume of a solution of the PCR amplification product was adjusted to 50 μl. An aliquot of the obtained solution of the PCR amplification product 1 that had been purified was subjected to electrophoresis according to a standard method to confirm that the desired PCR product was synthesized.

II. Production of DNA microarray

(1) Design of probe

Three types of probes were designed for the PCR product 1 described above. The probes were designed with consideration given to sequence, GC%, and melting temperature (Tm value) in the same way as the design of primers so that each probe can specifically recognize the designed partial nucleotide sequence. In this design of probes, a DNA strand extending from the primer R1 would hybridize with the probe to form a hybrid.

The sequence of each probe was designed by the adjustment of nucleotide length, etc., with consideration given to the stability of a hybrid.

The nucleotide sequences and Tm values of the designed probes are shown in Table 4.

Table 4

| SEQ ID No: | Probe | Base length | Sequence | Tm |
|---|---|---|---|---|
| SEQ ID No.3 | P1 | 60 | 5' TTTTATGGTGCACTCTCAGTACAATCTGCTCTG ATGCCGCATAGTTAAGCCAGCCCCGAC 3' | 90.7 |
| SEQ ID No.4 | P2 | 40 | 5' AGTTGGGTGCACGAGTGGGTTAC ATCGAACTGGATCTCAA 3' | 86.7 |
| SEQ ID No.5 | P3 | 25 | 5' GATAAAGTTGCAGGACCACTTCTGC 3' | 75.5 |

In a hybrid between a strand to be detected (a strand extending from R1) in the PCR product 1 and each probe, the length of a strand located upstream of the hybrid portion on the target strand is designated as L1 and the length of a strand located downstream thereof is designated as L2. The values of L1 and L2 of each probe for the PCR product are shown in Table 5.

Table 5

|  | L1 | L2 |
| --- | --- | --- |
| P1 | 1022 | 242 |
| P2 | 544 | 740 |
| P3 | 35 | 1264 |

(2) Synthesis of probe and production of DNA microarray

The synthesis of probes and the preparation of a DNA microarray are performed according to the DNA microarray production method disclosed by Canon.

That is, for a substrate process, silica glass was treated with a silane coupling agent and bound with EMCS to thereby introduce a maleimide group to the surface of the silica glass. For the synthesis of probes, each probe where a thiol group was introduced into the 5' end thereof was synthesized and purified by HPLC.

For producing a DNA microarray, a modification of a bubble jet printer (trade name: BJF-850, manufactured by Canon) was used to produce a DNA microarray in which each of the probes was spotted at 16 spots/substrate onto the glass substrate (size: 25 mm wide $\times$ 75 mm long $\times$ 1 mm tall).

III. Hybridization reaction

The DNA microarray produced in II and the PCR amplification product 1 produced as a sample nucleic acid in I were used to perform hybridization on the microarray.

(1) Blocking of DNA microarray

BSA (bovine serum albumin Fraction V, manufactured by Sigma) was dissolved in 100 mM NaCl/10 mM phosphate buffer so that the concentration was brought up to 1% by weight. The DNA microarray produced in II was soaked in this solution at room temperature for 2 hours to block the surface of the glass substrate. After the completion of blocking, the DNA microarray was washed with a 2xSSC solution (300 mM NaCl and 30 mM sodium citrate (trisodium citrate dihydrate, $C_6H_5Na_3.2H_2O$), pH 7.0) containing 0.1% by weight of SDS (sodium dodecyl sulfate) and subsequently rinsed with pure water. The DNA microarray was then dewatered with a spin dryer.

(2) Preparation of hybridization solution

Using 2 $\mu$l of a solution of the PCR amplification product, a hybridization solution was prepared so that the final concentration was brought up to the composition described below.

<Composition of hybridization solution>

6xSSPE/10% formamide/solution of PCR amplification product (6xSSPE: 900 mM NaCl, 60 mM $NaH_2PO_4.H_2O$ and 6 mM EDTA, pH 7.4)

(3) Hybridization

The dewatered DNA microarray was loaded in a hybridization apparatus (Genomic Solutions Inc. Hybridization Station), and the hybridization solution having the above-described composition was used to perform hybridization reaction according to the procedures and conditions below.

<Condition and procedure of hybridization>

The above-described hybridization solution was heated to 65°C and held for 3 minutes. The resulting hybridization solution was further held at 92°C for 2 minutes and subsequently at 55°C for 4 hours. Thereafter, the DNA microarray was washed at 25°C with 2xSSC and 0.1% SDS. After being further washed at 20°C with 2xSSC, the DNA microarray was rinsed with pure water according to a routine manual, if necessary, and dried by dewatering with a spin drier.

Table 6

| Step | Operation | Operational procedure and condition |
|---|---|---|
| 1 | Reaction | 65°C, 3 min. -> 92°C, 2 min. -> 55°C, 4 h. |
| 2 | Wash 1 | 2×SSC/0.1% SDS at 25°C |
| 3 | Wash 2 | 2×SSC at 20°C |
| 4 | Rinse for surface | $H_2O$ (manual rinsing) |
| 5 | Dry | Spin dry |

(4) Fluorescence Measurement

After the completion of hybridization reaction, the DNA microarray dried with a spin drier was measured for fluorescence derived from a hybrid using a DNA microarray fluorescence detector (Genepix 4000B, manufactured by Axon). The Result of measurement for each probe is shown in Table 7 below.

For calculating intensity, the fluorescent intensity observed in a portion without the spotted probe DNA on the DNA microarray was subtracted as a background value from the apparent fluorescent intensity of each probe spot. The obtained value was used as an actual value. Measurement was performed twice and its average is shown.

Table 7

| Probe | Fluorescent intensity |
|---|---|
| P1 | 2423 |
| P2 | 2614 |
| P3 | 4054 |

In this Example, three probes were assigned to domains to be detected that differ in hybrid stability. However, because a probe sequence was designed in consideration of the ratio of L1/L2 for each probe, the values of fluorescent intensity were obtained at almost the same level in four digits ranging from 2423 to 4054 in spite of the different level of hybrid stability.

(Example 2)

(1) Design of Probe

One type of probe was newly designed for the PCR product 1 synthesized in Example 1. The probe newly designed is a probe that is designed to detect a strand extending from the primer F1 in a portion corresponding to the P1 domain in Example 1. The probe was designed with consideration given to sequence, GC%, and melting temperature (Tm value) so that the probe can specifically recognize the designed partial nucleotide sequence. The nucleotide sequence and Tm value of the designed probe are shown in Table 8.

Table 8

| SEQ ID No: | Probe | Base length | Sequence | Tm |
|---|---|---|---|---|
| SEQ ID No:6 | P4 | 25 | 5' GCAGATTGTACTGAGAGTGCACCAT 3' | 76.4 |

The values of L1 and L2 are shown in Table 9 as in Example 1.

Table 9

| | L1 | L2 |
|---|---|---|
| P4 | 246 | 1053 |

(2) From production of DNA microarray through Hybridization

The synthesis of the probe, the production of a DNA microarray, and hybridization using the identical PCR product 1 were performed in the same way as Example 1 except that the probe P3 that was designed and used in Example 1 in addition to the probe P4 was spotted on the DNA microarray.

(3) Fluorescence measurement.

The result of measuring fluorescent intensity in the same way as Example 1 is shown in Table 10 below.

Table 10

| Probe | Fluorescent intensity |
|---|---|
| P3 | 2830 |
| P4 | 2520 |

In this Example, two probes were assigned to domains to be detected that differ in hybrid stability. Because the probe P4 of the domain having low hybrid stability was assigned to the complementary strand, hybrid stability was rendered high. As a result, the fluorescent intensity was obtained at almost the same level as the probe P3 even though the Tm value of the probe P4 is lower than that of the probe P1.

(Comparative Example 1)

(1) Design of probe

Two types of probes P5 and P6 were newly designed for the PCR product 1 synthesized in Example 1. The probes newly designed are probes that are designed to detect a strand extending from the primer R1 in portions corresponding to the P1 and P2 domains in Example 1. Both of two probes are designed to have the same binding strength (Tm value) as the probe P3. The nucleotide sequences and Tm values of the designed probes are shown in Table 11.

Table 11

| SEQ ID No: | Probe length | Base length | Sequence | Tm |
|---|---|---|---|---|
| SEQ ID No.7 | P5 | 25 | 5' ATGGTGCACTCTCAGTACAATCTGC3' | 76.4 |
| SEQ ID No.8 | P6 | 25 | 5' GTGGGTTACATCGAACTGGATCTCA3' | 75.7 |

The values of L1 and L2 are shown in Table 12 as in Example 1.

Table 12

| | L1 | L2 |
|---|---|---|
| P5 | 1053 | 246 |
| P6 | 545 | 754 |

(2) From production of DNA microarray through Hybridization

The synthesis of the probes, the production of a DNA microarray, and hybridization using the identical PCR product 1 were performed in the same way as Example 1 except that the probe P3 that was designed and used in Example 1 in addition to the probes P5 and P6 was spotted on the DNA microarray.

(3) Fluorescence measurement

The result of measuring fluorescent intensity in the same way as Example 1 is shown in Table 13 below.

Table 13

| Probe | Fluorescent intensity |
|---|---|
| P5 | 168 |
| P6 | 901 |
| P3 | 4054 |

In this Example, three probes were assigned to domains to be detected that differ in hybrid stability. All of the probes were set at the same level of binding strength (Tm value) without consideration given to hybrid stability. As a result, the value of fluorescent intensity obtained from each probe reflects hybrid stability and greatly differs among the probes in spite of the use of the identical sample.

**Claims**

1. A method for detecting a target single-stranded nucleic acid by utilizing a probe set comprising at least two probes each of which has a specific base sequence for the target single-stranded nucleic acid wherein a first probe and a second probe selected from the at least two probes satisfy the conditions:

   that the first probe has a base sequence which specifically binds to a first part of the single-stranded nucleic acid under a stringent condition;
   that the second probe has a base sequence which specifically binds to a second part of the single-stranded nucleic acid under the stringent condition; and
   that the first part is positioned farther away from 5'-terminal of the target single-stranded nucleic acid than the second part;
   **characterized in that** the Tm value of the first probe is set to be higher than that of the second probe such that the same levels of hybrid stability and hence the same detection sensitivities for the hybrids formed by the probes and the single-stranded nucleic acid are attained, thereby compensating generally lower hybrid stability at greater distance from the 5'terminal of the target nucleic acid.

2. The method according to claim 1, wherein the probes are DNAs.

3. The method according to claim 1 or claim 2, wherein a difference in Tm value between the probes comprising the probe set is set by setting the length of the nucleotide sequence of each of the probes.

4. The method according to claim 1 or claim 2, wherein a difference in Tm value between the probes comprising the probe set is set by setting the GC% of the nucleotide sequence of each of the probes.

5. The method according to claim 1 or claim 2, wherein a difference in Tm value between the probes comprising the probe set is set by the use of a Tm value calculated by the Nearest neighbor method based on the nucleotide sequence of each of the probes.

6. The method according to claim 1 or claim 2, wherein a difference in Tm value between the probes comprising the probe set is set by the use of a Tm value calculated by the Wallace method based on the nucleotide sequence of each of the probes.

7. The method according to claim 1 or claim 2, wherein a difference in Tm value between the probes comprising the probe set is set by the use of a Tm value calculated by the GC% method based on the nucleotide sequence of each of the probes.

8. The method according to claim 1, wherein the probes are PNAs.

9. The method according to claim 1, wherein the first and second probes, when respectively binding to the target single-stranded nucleic acid forming a hybrid, satisfy condition (1)

$$L1 \leq L2 \qquad (1)$$

   wherein L1 is the number of bases in a single strand portion including the 5'-terminal of the strand to be detected flanking the end of the double strand portion of the formed hybrid, and L2 is the number of bases in a single strand portion including the 3'-terminal of the strand to be detected flanking the end of the double strand portion of the formed hybrid.

10. A method for detecting two different domains in a target single-stranded nucleic acid of which the first domain is located on the 5'-terminal side of the target strand and the second domain is located on the 3'-terminal side of the target strand,
   **characterized by** utilizing the target single-stranded nucleic acid, a complementary strand thereof, and a probe set comprising at least two-probes, wherein
the first probe has a base sequence which specifically binds to said first domain under a stringent condition;
the second probe has a base sequence which specifically binds to the complement of said second domain in the complementary strand under the stringent condition; and
each of the first and second probes satisfies condition (1) when respectively binding to the target or complementary single-stranded nucleic acid and forming a respective hybrid,

$$L1 \leq L2 \qquad (1)$$

where L1 is the number of bases in a single strand portion including the 5'-terminal of the strand flanking the end of the double strand portion of the respective hybrid, and L2 is the number of bases in a single strand portion including the 3'-terminal of the strand flanking the end of the double strand portion of the respective hybrid thereby compensating general lower hybrid stability at greater distance from the 5'-terminal of the target nucleïc acid.

11. The method according to claim 10, wherein the probes are DNAs.

12. The method according to claim 10, wherein the probes are PNAs.

13. The method according to any of claim 1 to claim 12, wherein the probes are immobilized on distinguishably different domains of a solid phase, respectively.

14. The method according to claim 13, wherein each of the probes comprising the probe set is immobilized on the solid phase via a fixing site introduced into the 5'-terminal of the probe.

15. The method according to claim 13, wherein each of the probes comprising the probe set is immobilized on the solid phase via a fixing site introduced into the 3'-terminal of the probe.

16. The method according to claim 13, wherein each of the probes comprising the probe set is immobilized on the solid phase via a fixing site inserted into the sequence of the probe.

17. The method according to any of claim 13 to claim 16, wherein the probe substrate is a planar microarray.

**Patentansprüche**

1. Verfahren zum Nachweisen einer einzelsträngigen Zielnucleinsäure durch Verwendung eines Sondensatzes, der mindestens zwei Sonden umfasst, von denen jede eine spezifische Basensequenz für die einzelsträngige Zielnucleinsäure hat, wobei eine erste Sonde und eine zweite Sonde, die aus den zumindest zwei Sonden ausgewählt sind, die folgenden Bedingungen erfüllen:

   die erste Sonde hat eine Basensequenz, die sich unter strengen Bedingungen spezifisch an einen ersten Teil der einzelsträngigen Nucleinsäure bindet;
   die zweite Sonde hat eine Basensequenz, die sich unter den strengen Bedingungen spezifisch an einen zweiten Teil der einzelsträngigen Nucleinsäure bindet; und
   der erste Teil befindet sich weiter vom 5'-Ende der einzelsträngigen Zielnucleinsäure entfernt als der zweite Teil;
   **dadurch gekennzeichnet, dass**
   der Tm-Wert der ersten Sonde so eingestellt wird, dass er höher als der der zweiten Sonde wird, so dass die gleichen Werte der Hybridstabilität und folglich die gleichen Nachweisempfindlichkeiten für die von den Sonden und der einzelsträngigen Nucleinsäure gebildeten Hybride erreicht werden, wodurch die im allgemeinen geringere Hybridstabilität in einem größeren Abstand vom 5'-Ende der Zielnucleinsäure kompensiert wird.

2. Verfahren nach Anspruch 1, wobei
   die Sonden DNA sind.

3. Verfahren nach Anspruch 1 oder 2, wobei
   der Unterschied zwischen den Tm-Werten der Sonden, die der Sondensatz umfasst, durch Einstellen der Länge der Nucleotidsequenz jeder der Sonden festgelegt wird.

4. Verfahren nach Anspruch 1 oder 2, wobei
der Unterschied zwischen den Tm-Werten der Sonden, die der Sondensatz umfasst, durch Einstellen des Wertes GC% der Nucleotidsequenz jeder der Sonden festgelegt wird.

5. Verfahren nach Anspruch 1 oder 2, wobei
der Unterschied zwischen den Tm-Werten der Sonden, die der Sondensatz umfasst, durch Verwendung eines Tm-Wertes festgelegt wird, der durch die Nearest-Neighbor-Methode auf der Basis der Nucleotidsequenz jeder der Sonden berechnet wird.

6. Verfahren nach Anspruch 1 oder 2, wobei
der Unterschied zwischen den Tm-Werten der Sonden, die der Sondensatz umfasst, durch Verwendung eines Tm-Wertes festgelegt wird, welcher durch die Wallace-Methode auf der Basis der Nucleotidsequenz jeder der Sonden berechnet wird.

7. Verfahren nach Anspruch 1 oder 2, wobei
der Unterschied zwischen den Tm-Werten der Sonden, die der Sondensatz umfasst, durch Verwendung eines Tm-Wertes festgelegt wird, der durch die GC%-Methode auf der Basis der Nucleotidsequenz jeder der Sonden berechnet wird.

8. Verfahren nach Anspruch 1, wobei
die Sonden PNA sind.

9. Verfahren nach Anspruch 1, wobei
die erste und die zweite Sonde, wenn sie sich je entsprechend an die einzelsträngige Zielnucleinsäure binden, womit ein Hybrid entsteht, die Bedingung (1) erfüllen:

$$L1 \le L2 \qquad (1)$$

worin stehen:

L1 für die Anzahl der Basen in einem einzelsträngigen Abschnitt, der das 5'-Ende des nachzuweisenden Stranges einschließt, welcher das Ende des doppelsträngigen Abschnittes des erzeugten Hybrids flankiert, und
L2 für die Anzahl der Basen in einem einzelsträngigen Abschnitt, der das 3'-Ende des nachzuweisenden Stranges einschließt, welcher das Ende des doppelsträngigen Abschnittes des erzeugten Hybrids flankiert.

10. Verfahren zum Nachweisen von zwei unterschiedlichen Domänen in einer einzelsträngigen Zielnucleinsäure, bei der die erste Domäne auf der 5'-terminalen Seite des Zielstranges gelegen ist, und die zweite Domäne auf der 3'-terminalen Seite des Zielstranges,
**gekennzeichnet durch**
eine Verwendung der einzelsträngigen Zielnucleinsäure, eines dazu komplementären Stranges und eines Sondensatzes, der mindestens zwei Sonden umfasst, wobei
die erste Sonde eine Basensequenz hat, welche sich unter strengen Bedingungen spezifisch an die erste Domäne bindet;
die zweite Sonde eine Basensequenz hat, welche sich unter den strengen Bedingungen spezifisch an das Komplement der zweiten Domäne in dem komplementären Strang bindet; und
eine jede der ersten und zweiten Sonde die Bedingung (1), wenn sie sich entsprechend an das Ziel oder die komplementäre einzelsträngige Nucleinsäure binden und ein entsprechendes Hybrid bilden, erfüllen

$$L1 \le L2 \qquad (1)$$

worin stehen:

L1 für die Anzahl der Basen in einem einzelsträngigen Abschnitt, der das 5'-Ende des Stranges einschließt, welcher das Ende des doppelsträngigen Abschnittes des entsprechenden Hybrids flankiert, und

L2 für die Anzahl der Basen in einem einzelsträngigen Abschnitt, der das 3'-Ende des Stranges einschließt, welcher das Ende des doppelsträngigen Abschnittes des entsprechenden Hybrids flankiert, wodurch die im allgemeinen geringere Hybridstabilität in einem größeren Abstand vom 5'-Ende der Zielnucleinsäure kompensiert wird.

11. Verfahren nach Anspruch 10, wobei
die Sonden DNA sind.

12. Verfahren nach Anspruch 10, wobei
die Sonden PNA sind.

13. Verfahren nach einem der Ansprüche 1 bis 12, wobei
die Sonden je auf erkennbar unterschiedlichen Domänen einer festen Phase immobilisiert werden.

14. Verfahren nach Anspruch 13, wobei
jede der Sonden, die der Sondensatz umfasst, über einen Fixierungsort, der in das 5'-Ende der Sonde eingeführt ist, auf der festen Phase immobilisiert wird.

15. Verfahren nach Anspruch 13, wobei
jede der Sonden, die der Sondensatz umfasst, über einen Fixierungsort, der in das 3'-Ende der Sonde eingeführt ist, auf der festen Phase immobilisiert wird.

16. Verfahren nach Anspruch 13, wobei
jede der Sonden, die der Sondensatz umfasst, über einen Fixierungsort, der in die Sequenz der Sonde insertiert ist, auf der festen Phase immobilisiert wird.

17. Verfahren nach einem der Ansprüche 13 bis 16, wobei
das Sondensubstrat eine planare Mikroarray ist.


**Revendications**

1. Méthode pour détecter un acide nucléique monocaténaire cible en utilisant un ensemble de sondes comprenant au moins deux sondes dont chacune a une séquence de bases spécifique pour l'acide nucléique monocaténaire cible, dans laquelle une première sonde et une seconde sonde choisies parmi lesdites au moins deux sondes satisfont les conditions suivantes :

la première sonde a une séquence de bases qui se lie spécifiquement à une première partie de l'acide nucléique monocaténaire dans des conditions drastiques ;
la seconde sonde a une séquence de bases qui se lie spécifiquement à une seconde partie de l'acide nucléique monocaténaire dans les conditions drastiques ; et
la première partie est positionnée plus loin de l'extrémité 5'-terminale de l'acide nucléique monocaténaire cible, par rapport à la seconde partie ;
**caractérisée en ce que** la valeur de Tm de la première sonde est choisie de manière à être supérieure à celle de la seconde sonde afin que les mêmes valeurs de stabilité d'hybride et donc les mêmes sensibilités de détection pour les hybrides formés par les sondes et l'acide nucléique monocaténaire soient obtenues, ce qui compense la stabilité d'hybride généralement plus faible à une plus grande distance de l'extrémité 5'-terminale de l'acide nucléique cible.

2. Méthode suivant la revendication 1, dans laquelle les sondes sont des ADN.

3. Méthode suivant la revendication 1 ou la revendication 2, dans laquelle une différence de valeurs de Tm entre les sondes constituant l'ensemble de sondes est établie en fixant la longueur de la séquence de nucléotides de chacune des sondes.

4. Méthode suivant la revendication 1 ou la revendication 2, dans laquelle une différence de valeurs de Tm entre les sondes constituant l'ensemble de sondes est établie en fixant les % de GC de la séquence de nucléotides de chacune des sondes.

5. Méthode suivant la revendication 1 ou la revendication 2, dans laquelle une différence de valeurs de Tm entre les sondes constituant l'ensemble de sondes est établie par l'utilisation d'une valeur de Tm calculée par la méthode du voisin le plus proche basée sur la séquence de nucléotides de chacune des sondes.

6. Méthode suivant la revendication 1 ou la revendication 2, dans laquelle une différence de valeurs de Tm entre les sondes constituant l'ensemble de sondes est établie par l'utilisation d'une valeur de Tm calculée par la méthode de Wallace basée sur la séquence de nucléotides de chacune des sondes.

7. Méthode suivant la revendication 1 ou la revendication 2, dans laquelle une différence de valeurs de Tm entre les sondes constituant l'ensemble de sondes est établie par l'utilisation d'une valeur de Tm calculée par la méthode de % de GC basée sur la séquence de nucléotides de chacune des sondes.

8. Méthode suivant la revendication 1, dans laquelle les sondes sont des PNA.

9. Méthode suivant la revendication 1, dans laquelle les première et seconde sondes, lorsqu'elles se lient respectivement à l'acide nucléique monocaténaire cible en formant un hybride, satisfont la condition (1)

$$L1 \leq L2$$

dans laquelle L1 représente le nombre de bases dans une portion monocaténaire comprenant l'extrémité 5'-terminale du brin à détecter flanquant l'extrémité de la portion bicaténaire de l'hybride formé, et L2 représente le nombre de bases dans une portion monocaténaire comprenant l'extrémité 3'-terminale du brin à détecter flanquant l'extrémité de la portion bicaténaire de l'hybride formé.

10. Méthode pour détecter deux domaines différents dans un acide nucléique monocaténaire cible dont le premier domaine est situé du côté 5'-terminal du brin cible et le second domaine est situé du côté 3'-terminal du brin cible, **caractérisée par** l'utilisation de l'acide nucléique monocaténaire cible, d'un brin complémentaire de celui-ci, et d'un ensemble de sondes comprenant au moins deux sondes, dans laquelle
la première sonde a une séquence de bases qui se lie spécifiquement audit premier domaine dans des conditions drastiques ;
la seconde sonde a une séquence de bases qui se lie spécifiquement au complément dudit second domaine dans le brin complémentaire dans les conditions drastiques ; et
chacune des première et seconde sondes satisfait la condition (1) lors de la liaison respectivement à l'acide nucléique monocaténaire cible ou complémentaire et la formation d'un hybride respectif,

$$L1 \leq L2$$

où L1 représente le nombre de bases dans une portion monocaténaire comprenant l'extrémité 5'-terminale du brin flanquant l'extrémité de la portion bicaténaire de l'hybride respectif, et L2 représente le nombre de bases dans une portion monocaténaire comprenant l'extrémité 3'-terminale du brin flanquant l'extrémité de la portion bicaténaire de l'hybride respectif, ce qui compense la stabilité d'hybride généralement plus faible à une distance plus grande de l'extrémité 5'-terminale de l'acide nucléique cible.

11. Méthode suivant la revendication 10, dans laquelle les sondes sont des ADN.

12. Méthode suivant la revendication 10, dans laquelle les sondes sont des PNA.

13. Méthode suivant l'une quelconque des revendications 1 à 12, dans laquelle les sondes sont immobilisées sur des domaines distincts différenciables d'une phase solide, respectivement.

14. Méthode suivant la revendication 13, dans laquelle chacune des sondes constituant l'ensemble de sondes est immobilisée sur la phase solide par un site de fixation introduit dans l'extrémité 5'-terminale de la sonde.

15. Méthode suivant la revendication 13, dans laquelle chacune des sondes constituant l'ensemble de sondes est immobilisée sur la phase solide par un site de fixation introduit dans l'extrémité 3'-terminale de la sonde.

16. Méthode suivant la revendication 13, dans laquelle chacune des sondes constituant l'ensemble de sondes est immobilisée sur la phase solide par un site de fixation inséré dans la séquence de la sonde.

17. Méthode suivant l'une quelconque des revendications 13 à 16, dans laquelle le substrat de sonde est un micro-réseau plan.

# FIG. 1

# FIG. 2

# FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6410229 B
- JP 2003324647 A
- JP H11187900 B

### Non-patent literature cited in the description

- **Miguel Ángel Reyes-López et al.** Fingerprinting of prokaryotic 16S rRNA genes using oligodeoxyribonucleotide microarrays and virtual hybridization. *NUCLEIC ACIDS RESEARCH,* 2003, vol. 31 (2), 779-789
- **Martin Huber et al.** Accessing Single Nucleotide Polymorphisms in Genomic DNA by Direct Multiplex Polymerase Chain Reaction Amplification on Oligonucleotide Microarrays. *ANALYTICAL BIOCHEMISTRY,* 2002, vol. 303 (1), 25-33
- **Breslauer K.J. ; Frank R. ; Blocker H. ; Markey L.A.** Predicting DNA duplex stability from the nucleotide sequence. *Proc. Natl. Acad. Sci. USA,* 1986, vol. 83, 3746-3750
- **Freier S.M. Kierzek R. ; Jaeger J.A. ; Sugimoto N. ; Caruthers M.H. ; Nielson T. ; Turner D.H.** Improved free-energy parameters for predictions of RNA duplex stability. *Proc. Natl. Acad. Sci. USA,* 1986, vol. 83, 9373-9377
- **Schildkraut C. ; Lifson S.** Dependence of the melting temperature of DNA on salt concentration. *Biopolymers,* 1965, vol. 3, 195-208
- **Wallace, R.B. ; Shaffer, J. ; Murphy R.F. ; Bonner, J. ; Hirose, T. ; Itakura, K.** *Nucleic Acids Res.,* 1979, vol. 6, 3543
- **Schildkraut C. ; Lifson S.** Dependence of the Melting Temperature of DNA on Salt Concentration. *BIOPOLYMERS,* 1965, vol. 3, 195-208
- **W. Rychlik.** Optimization of the annealing temperature for DNA amplification in vitro. *Nucleic Acids Res.,* 1990, vol. 18 (21), 6409-6412